(19) Europäisches Patentamt
European Patent Office
Office européen des brevets

(11) Publication number: **0 194 862 B1**

# (12) EUROPEAN PATENT SPECIFICATION

(45) Date of publication of patent specification: **11.09.91** (51) Int. Cl.$^5$: **C07C 71/00**

(21) Application number: **86301748.9**

(22) Date of filing: **11.03.86**

## (54) Process for the preparation of fluoroxy halo compounds.

(30) Priority: **11.03.85 IT 1984785**

(43) Date of publication of application:
**17.09.86 Bulletin 86/38**

(45) Publication of the grant of the patent:
**11.09.91 Bulletin 91/37**

(84) Designated Contracting States:
**AT BE CH DE FR GB LI NL SE**

(56) References cited:
**GB-A- 2 092 137**
**US-A- 3 442 927**

**JOURNAL OF THE AMERICAN CHEMICAL SOCIETY, vol. 88:19, 5th October 1966, pages 4531-4532; J.K. RUFF et al.: "A simple synthesis of fluoroxyperfluoroalkyl compounds"**

**JOURNAL OF THE AMERICAN CHEMICAL SOCIETY, vol. 89:12, 7th June 1967, pages 2841-2843; M. LUSTIG et al.: "The catalytic addition of fluorine to a carbonyl group. Preparation of fluoroxy compounds"**

(73) Proprietor: **AUSIMONT S.p.A.**
**31, Foro Buonaparte**
**I-20121 Milano(IT)**

(72) Inventor: **Guglielmo, Giorgio**
**8, via Cavallotti**
**I-21013 Gallarate Varese(IT)**
Inventor: **Conte, Lino**
**15, via Padre Leopoldo**
**I-35028 Piove Di Sacco Padova(IT)**
Inventor: **Carlini, Filippo Maria**
**66 Corso Padova**
**36100 Vicenza(IT)**

(74) Representative: **Whalley, Kevin et al**
**MARKS & CLERK 57/60 Lincoln's Inn Fields**
**London WC2A 3LS(GB)**

EP 0 194 862 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention concerns a process for the preparation of fluoroxy-halo-compounds. More particularly the present invention refers to a continuous process for the preparation of fluoroxy-halo-compounds of the formula:

$(R)_nC(F)_m$-O-F (I)

wherein R is: an alkyl or cycloalkyl radical containing from 1 to 12 carbon atoms, either partially or fully halogenated with bromine, chlorine, and/or fluorine or R is a perfluoromonoether or perfluoropolyether radical containing from 1 to 12 carbon atoms; n is 1 or 2; m is equal to 3-n; it being understood that the value n = 2 comprises the compounds in which C is part of a cyclic ring.

Fluoroxy-halo-compounds comprised by the above indicated general formula (I) are quite well known in the literature and described in U.S. Patent US-A-3,442,927, by John R. Ruff et al. in J.A.C.S., 88:19, Oct. 5, 1966 pages nos. 4531-4532, and by Lustig et al. in J.A.C.S. 89:12, June 7, pages 2841-2843.

These compounds find a useful application as oxidizers, for example in the bleaching, as oxidizing agents in organic syntheses in various different applicational fields, as oxidizers in propellants, and as fluorinating agents.

Fluoroxy-halo-compounds have, so far, been considered difficult to produce, mainly because of their high instability.

Due to the high instability of the products, the various processes proposed for their preparation are conducted under such drastic and limitative conditions as not to find a practical industrial application.

In fact, operating according to the known processes, there are obtained very low yields (~ 2%), or it is necessary to operate at very low reaction temperatures, in general at -78° C, or the reaction is not selective and one gets the formation of by-products that subsequently have to be removed. At any rate, the known processes all operate in a discontinuous way and with very low quantities of reactant.

Thus, for instance, in J.A.C.S. 80:19 - Oct. 5, 1966 on pages 4531 to 4532 and in J.A.C.S. 89:12 - June 7, 1967 on pages 2841 to 2843 there is described the preparation of fluoroxyalkanes by the catalytic addition of fluorine on the double carbon/oxygen bond in a perfluorocarbonyl compound according to the scheme:

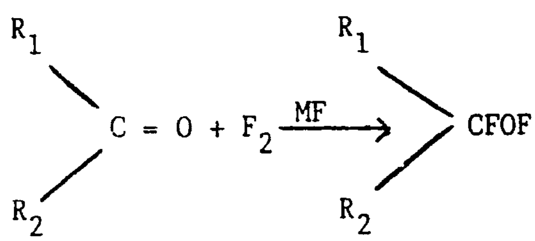


wherein: $R_1$ and $R_2$ may be, independently from each other, fluorine or a perfluoroalkyl radical while M is K, Na, Rb or Cs.

The reaction is conducted in a discontinuous way, on small quantities, at a low temperature (-78° C), at a reduced absolute pressure of 10-20 kPa, and in long times. Although the process allows one to obtain high yields (up to 98%), the quantity of obtainable fluoroxy-alkanes does not exceed 10 mM (millimoles) per charge (batch).

US-A-3,442,927 describes a process for the production of fluoroxy-compounds by direct reaction in a discontinuous way of fluorine with oxygen-containing compounds whose oxygen is directly bound to carbon, at temperatures comprised between -100° C and +50° C, in which process the desired products are isolated from the reaction mixture by fractioned condensation using separators refrigerated with dry ice, liquid air, ice-salt for the low-boiling products, and other suitable temperature conditions for the high-boiling liquids. The fluorine and the other gases are introduced into the reaction medium, under a slight pressure, and the reaction time is between 10 minutes and 12 h.

The main drawbacks of this process consist in the lack of selectivity, in low yields, and in the necessity of carrying out the fractioned separation of the products obtained, at a low temperature and under perfectly clean conditions in order to avoid explosive decomposition of the fluoroxy-compounds which, as is well known, is provoked by traces of pollutants.

Moreover, also in this process the reaction is conducted in a discontinuous way and on small quantities of product.

Thus, what is desired is a process for the preparation of fluoroxy-compounds that will not display the above mentioned drawbacks, more particularly, an industrial process for the preparation of fluoroxy-compounds. The industrial process should allow one to produce in a selective way and with high yields, the desired fluoroxy compound. It should also be possible to obtain fluoroxy-compounds with a high productivity and high purity without requiring subsequent isolation and/or manipulation, so as to avoid the risks and limitations connected with their high instability.

We have now found, surprisingly, that these aims can be achieved by carrying out a direct reaction between fluorine and an organic compound having a molecular structure in which at least one atom of oxygen is directly bound to a carbon atom in the carbonylic form, in the presence of a fluorination catalyst, in a gaseous phase and in such conditions as to obtain the fluoroxy-halo-compound in the gaseous state, at an absolute pressure comprised between 50 and 800 kPa, at a temperature greater than -50 °C and up to 150 °C, under conditions of continuous feeding of the reactants and continuous removal of the reaction product, so that the mean dwell time of the reactants in the reaction medium is less than 10 minutes, and under conditions of removal of the reaction heat, in order to maintain the temperature at values of not more than 150 °C.

The gaseous mixture fed to the reaction may contain a diluent. The operational conditions of the reaction, including the possible dilution conditions, are chosen in such a way that the starting carbonyl compound and the obtained fluoroxy-halo-compound are both in the gaseous state.

According to a preferred embodiment of the present invention, the fluorination reaction is conducted by continuously passing the reactants in a gaseous phase through a layer containing the catalyst and capable of ensuring the attainment of the required heat exchange.

The compound having a molecular structure in which at least one oxygen atom is directly linked to a carbon atom in a carbonylic form, may be more specifically represented by the formula:

$(R)_nC(X)_{m-1}O$ (II)

wherein:• O represents an oxygen atom directly linked to carbon, X represents fluorine or chlorine, while R, n, and m have the same values and meanings as indicated previously above.

When R contains H and/or Br, it is preferred to operate the reaction at a temperature not higher than 50 °C.

The compounds in which R represents a perfluoro-alkyl radical having from 1 to 5 carbon atoms or a perfluoromonoether or perfluoropolyether radical having from 1 to 5 carbon atoms are preferred compounds in the process of the present invention.

Examples illustrating the starting compounds to be submitted to the fluorination process according to the present invention are: fluoride and chloride of trifluoro-acetyl; fluoride and chloride of chloro,difluoro-acetyl; fluoride and chloride of trichloro-acetyl; fluoride and chloride of bromo,difluoro-acetyl; fluoride and chloride of penta fluoro-propionyl; fluoride and chloride of heptafluoro-butyryl; hexafluoroacetone; and perfluoroetoxy-difluoro-acetyl-fluoride, perfluorometoxy-difluoro-acetylfluoride, and a mixture thereof.

The reactants are continuously fed in, in gaseous phase, preferably under a slight pressure and in equimolar quantities, with flow rates for each reactant greater than $5.10^{-6}$ mol/h per gram of catalyst. Flow rates for each reactant that are between $10^{-4}$ and $10^{-1}$ mol/h per gram of catalyst are preferred.

The reactants are preferably diluted with an inert gas in order to obtain concentrations comprised between 5% and 70% by volume. As an inert gas there may be used any gas or vapour that is non-reactive with the reactants and the reaction products such as, for instance, nitrogen, argon, helium, tetrafluoromethane, dichlorotetrafluoroethanes, pentafluorochloroethane, difluorodichloromethane, perfluorocyclobutane, etc.

As a catalyst there may be used any kind of known fluorination catalyst, particularly caesium fluoride, rubidium fluoride, potassium fluoride, lithium fluoride, or sodium fluoride; a caesium fluoride is the preferred one.

In order to remove the reaction heat and achieve the required heat exchange, the process may be carried out in the presence of a metallic material substantially inert to fluorine, or carried out in a metal reactor having such a geometric configuration as to ensure the required heat exchange.

Amongst the metallic materials suited for the purpose we would cite before all and preferably copper and its alloys such as brass and Monel, although also other materials may be used.

The catalyst can be mixed with the metallic material, the material being in the form of chips (shavings), Raschig rings, or similar filling bodies or can be supported on the metallic material which is in one of the

abovesaid forms. It is also possible to use the catalyst both mixed with the metallic material and supported on it.

According to a preferred embodiment of the present invention, the catalyst is intermingled (mixed) with the metal material and/or supported on it, the metal material being in the form of shavings, Raschig rings or similar filling bodies, and the reactants, in gaseous phase and diluted in an inert gas, are made to continuously pass through a fixed layer of the metallic material catalyst, with such a flow speed as to maintain the dwell time in the reactor below 10 minutes, keeping the reaction temperature at a value between -20 °C and 100 °C, but preferably between 20 °C and 60 °C, while the absolute pressure is between 100 and 200 kPa.

In order to regulate (control) the reaction temperature, any known suitable means may be used, for instance a thermostatic bath.

The gaseous mixture that goes to the reaction should be well purified from HF and $H_2O$ and from the acid or dialcohol that can form by hydrolysis of the starting carbonylic compound. More precisely the inert gas should be purified from $H_2O$, the $F_2$ from HF, and the starting carbonylic compound from HF and from the possible acid or dialcohol.

The process of the present invention allows one to obtain the full conversion of the reactants with stoichiometric yields in a pure fluoroxy-compound which does not need any successive operations for its isolation or purification. The fluoroxy compounds obtained according to the process of the present invention find their useful application in condensation and/or fluorination reactions of the most various organic substrates (compounds) such as: olefines, unsaturated compounds, organic compounds having hydrogen or functional groups that can be substituted with fluorine, etc., or they may be decomposed with the formation of $COF_2$ and of the corresponding halogeno-compound.

In order to give a better understanding of the invention and its practical actuation, a series of illustrative but not limiting examples follow.

Example 1

Into a brass reactor, having a diameter of 50 mm and a useful holding capacity of 500 $cm^3$, completely filled with copper chips (shavings) intermingled with 300 g of CsF (dried at 250 °C in a nitrogen flow during 4 hours, milled, and screened in a dry box in order to obtain a granulometry between 250 and 500 micron), there was fed continuously a mixture of $N_2/F_2/CF_3COF$(purified from HF, $H_2O$, and the acid or dialcohol that can form by hydrolysis of the starting carbonylic compound) in a molar ratio equal to 3.75/1/1, at a global flow rate of 23 Nl/hr ($6.10^{-4}$ mol/h per g of catalyst for each reactant) and at an absolute pressure of 110 kPa. The temperature inside the reactor was kept at -10 °C by means of external cooling.

The gaseous mixture flowing out of the reactor, after analysis by IR spectrophotometry and iodometry, proved to consist of $N_2/CF_3CF_2OF$ in a molar ratio of 3.75/1, with a 100% conversion and a 100% yield of perfluoro-ethoxy-fluoride.

After 400 hours of operation, the conversion of the reactants and the yield remained unvaried.

On increasing the temperature inside the reactor to +20 °C or increasing the absolute pressure to 500 kPa, the conversions and the yields remained unvaried at the value of 100%.

Example 2

Into the reactor described in Example 1, there was continuously fed a gaseous mixture consisting of: $N_2/F_2/CF_3COF$ (purified as indicated above) in a molar ratio of 1/1/1, at a global flow rate of 12 Nl/h ($6.10^{-4}$ mol/h per gram of catalyst for each reactant) and at an absolute pressure of 110 kPa. The temperature inside the reactor was maintained at +20 °C by means of a thermostatically controlled bath. The gaseous mixture flowing out of the reactor, analysed by means of IR spectrophotometry and iodometry, proved to consist of $N_2/CF_3CF_2OF$ in a molar ratio of 1/1, with 100% conversion and yield.

Even after several weeks of operation, no variations in the conversion and/or yield could be noticed.

Example 3

Into the same reactor as that of Example 1, there was continuously fed a gaseous mixture of $N_2/F_2/CClF_2COF$ (purified as indicated above) in a molar ratio of 3.75/1/1, at a total flow rate of 23 Nl/h ($6.10^{-4}$ mol/h per gram of catalyst for each reactant) and at an absolute pressure of 110 kPa. The temperature inside the reactor was maintained at 20 °C. The gaseous mixture flowing out of the reactor, analyzed by means of IR spectrophotometry and iodometry, proved to consist of $N_2$ and $CClF_2$-$CF_2OF$ in a

molar ratio of 3.75/1, with a 100% conversion and a 100% yield of 2-chloro-tetrafluoro-ethoxy-fluoride. The yield and the conversion remained unvaried after several weeks.

Example 4

Into an AISI 316 reactor of 25 mm diameter and of 500 $cm^3$ holding capacity, completely filled with Raschig copper rings of 4 mm size, intermingled with 300 g of CsF (treated as Indicated in Example 1), was fed continuously a gaseous mixture of $N_2/F_2/CF_3COF$(purified as indicated above) in a molar ratio of 30/2/1, at a total flow rate of 16.5 Nl/h ($7.4.10^{-5}$ mol/h of $CF_3COF$ per gram of catalyst and $14.9.10^{-5}$ mol/h of fluorine per gram of catalyst), and at an absolute pressure of 110 kPa.

The temperature inside the reactor was kept at 135° C by means of a thermostatic bath. The outflowing gases were analyzed by IR spectrophotometry and iodometry, and they proved to consist of $N_2$ and $CF_3CF_2OF$ with complete conversion of $CF_3COF$ into $CF_3CF_2OF$.

Example 5

Into the reactor described in Example 4, was continuously fed a gaseous mixture consisting of $C_2ClF_5/F_2/CF_3COF$ (purified as indicated above) in a molar ratio of 1/1/1, at a total flow rate of 45 Nl/h ($2.2.10^{-3}$ mol/h per gram of catalyst for each reactant), and at an absolute pressure of 110 kPa. The temperature in the reactor was kept at 20° C by means of external cooling. The outflowing gaseous mixture, analyzed by means of IR spectrophotometry and iodometry, proved to consist of $C_2ClF_5/CF_3CF_2OF$ in a molar ratio equal to 1/1, with a 100% conversion and a 100% yield of pentafluoro-ethoxy-fluoride.

Example 6

Into the same reactor as that of Example 1, there was continuously fed a gaseous mixture of $N_2/F_2/CF_3COCl$ (purified as indicated above) in a molar ratio of 3.75/1.5/1, at a global flow rate of 25 Nl/h ($9.10^{-4}$ mol/h of fluorine per gram of catalyst and $6.10^{-4}$ mol/h of $CF_3COCl$ per gram of catalyst) and at an absolute pressure of 110 kPa. The temperature inside the reactor was maintained at 30° C by external cooling. The outflowing gaseous mixture, analyzed by means of IR spectrophotometry and iodometry and titration of chlorides, proved to consist of $N_2/CF_3CF_2OF/Cl_2$ in a molar ratio of 7.5/2/1, with a 100% conversion and a 100% yield of pentafluoro-ethoxy fluoride.

Example 7

Into the reactor of Example 1, there was continuously fed a gaseous mixture of $N_2/F_2/(CF_3)_2CO$ (purified as indicated above) in a molar ratio of 3.75/1/1, at a global flow rate of 23 Nl/h ($6.10^{-4}$ mol/h per gram of catalyst for each reactant), and at an absolute pressure of 110 kPa. The temperature inside the reactor was maintained at 20° C by external cooling.

The outflowing gaseous mixture, analyzed by IR spectrophotometry and iodometry, proved to consist of $N_2/(CF_3)_2$-CF-O-F in a molar ratio of 3.75/1, with a 100% conversion and yield.

Example 8

Into the reactor of Example 1, there was continuously fed a gaseous mixture of $N_2/F_2/CF_3$-$CF_2$-COF (purified as indicated above) in a molar ratio of 3.75/1/1, at a global flow rate of 23 Nl/h ($6.10^{-4}$ mol/h per gram of catalyst for each reactant), and at an absolute pressure of 110 kPa. The temperature inside the reactor was maintained at 20° C by external cooling. The outflowing gaseous mixture, analyzed by IR spectrophotometry and iodometry, proved to consist of $N_2/CF_3$-$CF_2$-$CF_2$-OF in a molar ratio of 3.75/1, with a 100% conversion and yield.

Example 9

Into the reactor of Example 1, there was continuously fed a gaseous mixture of

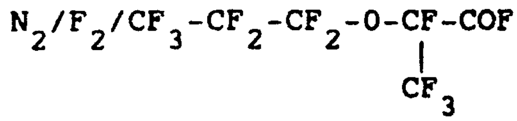

(purified as indicated above) in a molar ratio of 3.75/1/1, at a global flow rate of 23 Nl/h ($6.10^{-4}$ mol/h per gram of catalyst for each reactant), and at an absolute pressure of 110 kPa. The temperature inside the reactor was maintained at 20° C by external cooling. The outflowing gaseous mixture, analyzed by IR spectrophotometry and iodometry, proved to consist of

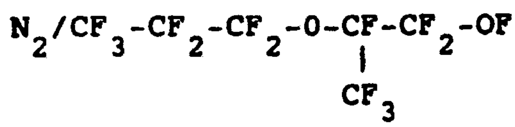

in a molar ratio of 3.75/1, with a 100% conversion and yield.

## Claims

1. A process for the preparation of a fluoroxy-halo-compound of the formula:

   $(R)_nC(F)_m$-O-F (I)

   wherein either R is an alkyl or cycloalkyl radical containing from 1 to 12 carbon atoms, partially or fully halogenated with bromine, chlorine, and/or fluorine or R is a perfluoromonoether or perfluoropolyether radical containing from 1 to 12 carbon atoms, n = 1 or 2, and m = 3-n, it being understood that when n is 2 the formula includes compounds in which C is part of a cyclic ring, the said fluoroxy-halo-compound being prepared by direct reaction, in the presence of a fluorination catalyst, between fluorine and an organic compound having a molecular structure in which at least one oxygen atom is directly linked to a carbon atom in the carbonylic form, characterized in that the reaction is conducted in gaseous phase and in such conditions as to obtain the fluoroxy-halo-compound in the gaseous state, at an absolute pressure of 50 to 800 kPa, at a temperature higher than -50° C and up to 150° C, under conditions of continuous feeding of the reactants and continuous removal of the reaction product, so that the dwell time of the reactants in the reaction medium is less than 10 minutes, and under conditions of removal of the reaction heat in order to prevent the temperature exceeding 150° C.

2. A process as claimed in claim 1, in which the said organic compound, having a molecular structure wherein at least one oxygen atom is directly linked to a carbon atom in the carbonylic form, is of the formula:

   $(R)_nC(X)_{m-1}O$ (II)

   wherein: O represents an oxygen atom directly linked to the carbon; X represents fluorine or chlorine; and R, m, and n have the same values and meanings as in formula (I).

3. A process as claimed in claim 1 or 2, in which R represents a perfluoro-alkyl radical having from 1 to 5 carbon atoms or a perfluoromonoether or perfluoropolyether radical having from 1 to 5 carbon atoms.

4. A process as claimed in any preceding claim, in which the reaction between the fluorine and the said organic compound, having a molecular structure in which at least one oxygen atom is directly bound to a carbon atom in the carbonylic form, is conducted by means of continuous passage of the reactants in gaseous phase through a fixed bed containing the catalyst and capable of ensuring the required thermal exchange.

5. A process as claimed in any preceding claim, conducted in the presence of a metallic material substantially inert to fluorine.

6. A process as claimed in claim 5 when dependent on claim 4, in which the catalyst is intermingled with the metallic material, the metallic material being in the form of chips, Raschig rings, or similar filling bodies, and/or is supported on the metallic material.

7. A process as claimed in claim 5 or 6, in which the metallic material is copper or an alloy thereof, e.g. brass or Monel.

8. A process as claimed in any preceding claim, in which each reactant is fed in with a flow rate greater than $5.10^{-5}$ mol/h per gram of catalyst.

9. A process as claimed in claim 8, in which the flow rate for each reactant is $10^{-4}$ to $10^{-1}$ mol/h per gram of catalyst.

10. A process as claimed in claim 1, in which the catalyst is intermingled with metallic material substantially inert to fluorine in the form of chips, Raschig rings or similar filling bodies, and/or supported on the metallic material, and the reactants, in gaseous phase and diluted in an inert gas, are continuously passed through a fixed layer of the metallic material/catalyst at a speed corresponding to a dwell time in the reactor below 10 minutes, while maintaining a reaction temperature of -20° C to +100° C, preferably 20° C to 60° C, while the absolute pressure is 100 to 200 kPa.

**Revendications**

1. Un procédé de préparation d'un dérivé fluoroxy halogéné répondant à la formule:

$(R)_nC(F)_m$-O-F (I)

dans laquelle:

- R représente un radical alkyle ou cycloalkyle renfermant de 1 à 12 atomes de carbone, soit partiellement, soit totalement halogéné par du brome, du chlore, et/ou du fluor, ou R représente un radical perfluoromonoéther ou perfluoropolyéther renfermant de 1 à 12 atomes de carbone;
- n est égal à 1 ou 2; et
- m est égal à 3-n;

étant entendu que lorsque n est égal à 2, la formule comprend des dérivés dans lesquels C fait partie d'un cycle, ce dérivé fluoroxy halogéné étant préparé par réaction directe, en présence d'un catalyseur de fluoration, entre du fluor et un dérivé organique dans la structure moléculaire duquel au moins un des atomes d'oxygène est directement lié à un atome de carbone sous la forme carbonylique, caractérisé en ce que la réaction est conduite en phase gazeuse et dans des conditions propres à obtenir le dérivé fluoroxy halogéné à l'état gazeux, la pression absolue étant comprise entre 50 et 800 kPa, la température étant supérieure à -50° C et pouvant atteindre jusqu'à 150° C, dans des conditions d'introduction des réactifs en continu et d'élimination en continu du produit de la réaction, de façon à ce que le temps de séjour des réactifs dans le milieu réactionnel soit inférieur à 10 minutes et dans des conditions d'élimination de la chaleur de réaction pour empêcher la température de dépasser 150° C.

2. Un procédé selon la revendication 1, caractérisé en ce que ce dérivé organique dans la structure moléculaire duquel au moins l'un des atomes d'oxygène est rattaché directement à un atome de carbone sous la forme carbonylique répond à la formule:

$(R)_nC(X)_{m-1}O$ (II)

dans laquelle:

- O représente un atome d'oxygène directement lié au carbone;
- X représente un atome de fluor ou de chlore alors que R, m, et n présentent les mêmes valeurs et les mêmes significations que dans la formule (I).

3. Un procédé selon l'une des revendications 1 ou 2, caractérisé en ce que R représente un radical perfluoroalkyle renfermant de 1 à 5 atomes de carbone ou un radical perfluoromonoéther ou perfluoropolyéther renfermant de 1 à 5 atomes de carbone.

**4.** Un procédé selon l'une quelconque des revendications précédentes dans lequel la réaction entre le fluor et ledit dérivé organique dans la structure moléculaire duquel au moins l'un des atomes d'oxygène est directement relié à un atome de carbone sous sa forme carbonylique, est conduite par passage en continu des réactifs en phase gazeuse à travers un lit fixe contenant le catalyseur et capable de garantir l'échange thermique convenable.

**5.** Un procédé selon l'une quelconque des revendications précédentes conduit en présence d'un matériau métallique sensiblement inerte au fluor.

**6.** Un procédé selon la revendication 5, prise en dépendance de la revendication 4, dans lequel le catalyseur est intermélangé avec le matériau métallique, le matériau métallique se trouvant sous la forme de copeaux, anneaux de Raschig ou corps de remplissage semblables et/ou il est supporté sur le matériau métallique.

**7.** Un procédé selon l'une des revendications 5 ou 6, caractérisé en ce que le matériau métallique est le cuivre ou l'un de ses alliages, par exemple le laiton ou le Monel.

**8.** Un procédé selon l'une quelconque des revendications précédentes, caractérisé en ce que chaque réactif est introduit à un débit supérieur à $5.10^{-6}$ moles/h par gramme de catalyseur.

**9.** Un procédé selon la revendication 8, caractérisé en ce que le débit de chaque réactif est compris entre $10^{-4}$ et $10^{-1}$ moles/h par gramme de catalyseur.

**10.** Un procédé selon la revendication 1, caractérisé en ce que le catalyseur est entremêlé de matériau métallique sensiblement inerte au fluor prenant la forme de copeaux, anneaux de Raschig ou corps de remplissage semblables et/ou supporté sur le matériau métallique, et les réactifs, en phase gazeuse et dilués dans un gaz inerte, passent en continu à travers une couche fixe du catalyseur à base de matériau métallique à une vitesse correspondant à un temps de séjour dans le réacteur inférieur à 10 mn, alors que l'on maintient une température de réaction comprise entre -20 et +100 °C, de préférence 20 à 60 °C, la pression absolue étant comprise entre 100 et 200 kPa.

## Patentansprüche

**1.** Verfahren zur Herstellung einer Fluoroxyhalogenverbindung der Formel:

$(R)_nC(F)_m$-O-F (I)

worin R entweder einen Alkyl- oder Cycloalkylrest mit 1 bis 12 Kohlenstoffatomen, der teilweise oder vollständig mit Brom, Chlor und/oder Fluor halogeniert ist, bedeutet oder R einen Perfluormonoether- oder Perfluorpolyetherrest mit 1 bis 12 Kohlenstoffatomen darstellt, n = 1 oder 2 und m = 3-n, mit der Maßgabe, daß wenn n = 2, die Formel Verbindungen einschließt, in denen C Teil eines cyclischen Rings ist,
wobei die Fluoroxyhalogenverbindung durch direkte Reaktion in Gegenwart eines Fluorierungskatalysators zwischen Fluor und einer organischen Verbindung, die eine Molekularstruktur aufweist, in der mindestens ein Sauerstoffatom direkt an ein Kohlenstoffatom in Carbonylform gebunden ist, hergestellt wird, dadurch gekennzeichnet, daß die Reaktion in der Gasphase unter Bedingungen, welche die Fluoroxyhalogenverbindung im gasförmigen Zustand ergeben, bei einem absoluten Druck von 50 bis 800 kPa, bei einer Temperatur höher als -50 °C und bis zu 150 °C unter den Bedingungen einer kontinuierlichen Zufuhr der Reaktanden und kontinuierlichen Entfernung des Reaktionsprodukts, so daß die Verweilzeit der Reaktanden in dem Reaktionsmedium weniger als 10 Minuten beträgt, und unter Bedingungen zur Ableitung der Reaktionswärme, um zu verhindern daß die Temperatur 150 °C übersteigt, durchgeführt wird.

**2.** Verfahren nach Anspruch 1, worin die organische Verbindung, die eine Molekularstruktur aufweist, in der mindestens ein Sauerstoffatom direkt an ein Kohlenstoffatom in Carbonylform gebunden ist, die Formel aufweist:

$(R)_nC(X)_{m-1}O$ (II)

worin O ein direkt an Kohlenstoff gebundenes Sauerstoffatom repräsentiert; X Fluor oder Chlor darstellt; und R, m und n dieselben Werte und Bedeutungen wie in Formel (I) haben.

**3.** Verfahren nach Anspruch 1 oder 2, worin R einen Perfluoralkylrest mit 1 bis 5 Kohlenstoffatomen oder einen Perfluormonoether- oder Perfluorpolyetherrest mit 1 bis 5 Kohlenstoffatomen darstellt.

**4.** Verfahren nach irgendeinem der vorhergehenden Ansprüche, worin die Reaktion zwischen Fluor und der organischen Verbindung, die eine Molekularstruktur aufweist, in der mindestens ein Sauerstoffatom direkt an ein Kohlenstoffatom in Carbonylform gebunden ist, durchgeführt wird, indem die Reaktanden in der Gasphase kontinuierlich durch ein Festbett geleitet werden, das den Katalysator enthält und in der Lage ist, den erforderlichen Wärmeaustausch zu gewährleisten.

**5.** Verfahren nach irgendeinem der vorhergehenden Ansprüche, das in Gegenwart eines metallischen Materials, welches im wesentlichen inert gegenüber Fluor ist, durchgeführt wird.

**6.** Verfahren nach Anspruch 5, wenn dieser von Anspruch 4 abhängig ist, worin der Katalysator mit dem metallischen Material vermischt ist, wobei das metallische Material in Form von Spänen, Raschigringen oder ähnlichen Füllkörpern vorliegt, und/oder vom metallischen Material getragen wird.

**7.** Verfahren nach Anspruch 5 oder 6, worin das metallische Material Kupfer oder dessen Legierungen wie Messing oder Monel darstellt.

**8.** Verfahren nach irgendeinem der vorhergehenden Ansprüche, worin jeder Reaktand mit einer Flußrate größer als $5x10^{-5}$ Mol/h pro Gramm Katalysator zugeführt wird.

**9.** Verfahren nach Anspruch 8, worin die Flußrate für jeden Reaktanden $10^{-4}$ bis $10^{-1}$ Mol/h pro Gramm Katalysator beträgt.

**10.** Verfahren nach Anspruch 1, worin der Katalysator mit metallischem Material vermischt ist, das im wesentlichen inert gegenüber Fluor ist und in der Form von Spänen, Raschigringen oder ähnlichen Füllkörpern vorliegt, und/oder vom metallischen Material getragen wird, und die Reaktanden in der Gasphase und mit einem Inertgas verdünnt, kontinuierlich durch eine feste Schicht aus metallischem Material/Katalysator mit einer Geschwindigkeit geleitet werden, die einer Verweilzeit im Reaktor unter 10 Minuten entspricht, wobei eine Reaktionstemperatur von -20°C bis 100°C, vorzugsweise 20°C bis 60°C, aufrechterhalten wird und der absolute Druck 100 bis 200 kPa beträgt.